(19) Europäisches Patentamt
European Patent Office
Office européen des brevets

(11) EP 3 479 822 B1

(12) **EUROPEAN PATENT SPECIFICATION**

(45) Date of publication and mention of the grant of the patent:
**07.06.2023 Bulletin 2023/23**

(21) Application number: **17820624.9**

(22) Date of filing: **28.06.2017**

(51) International Patent Classification (IPC):
***A61K 31/13*** *(2006.01)* ***A61K 31/4045*** *(2006.01)*
***A61K 9/20*** *(2006.01)* ***A61P 25/28*** *(2006.01)*
***A61K 9/00*** *(2006.01)*

(52) Cooperative Patent Classification (CPC):
(C-Sets available)
**A61K 9/2009; A61K 9/2013; A61K 9/2018; A61K 9/2027; A61K 9/2031; A61K 9/2054; A61K 31/13; A61K 31/4045; A61P 25/28** (Cont.)

(86) International application number:
**PCT/RU2017/000458**

(87) International publication number:
**WO 2018/004391 (04.01.2018 Gazette 2018/01)**

(54) **PHARMACEUTICAL COMPOSITION CONTAINING COMBINATION OF MEMANTINE AND MELATONIN**

PHARMAZEUTISCHE ZUSAMMENSETZUNG MIT EINER KOMBINATION AUS MEMANTIN UND MELATONIN

COMPOSITION PHARMACEUTIQUE CONTENANT UNE COMBINAISON DE MÉMANTINE ET DE MÉLATONINE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **29.06.2016 RU 2016125970**
**29.06.2016 RU 2016125973**

(43) Date of publication of application:
**08.05.2019 Bulletin 2019/19**

(73) Proprietor: **Ltd. "Valenta-Intellekt"**
**Moscow 119530 (RU)**

(72) Inventors:
- **SYROV, Kirill Konstantinovich**
**Moscow 121609 (RU)**
- **NESTERUK, Vladimir Viktorovich**
**Moscow 125171 (RU)**

(74) Representative: **Franco Martegani S.r.l.**
**Via Carlo Alberto, 41**
**20900 Monza (IT)**

(56) References cited:
**WO-A1-2013/176567**
**WO-A1-2015/060746**
**WO-A2-2008/005534**
**CN-B- 101 143 135**
**RU-C1- 2 557 960**
**RU-C2- 2 482 839**
**RU-C2- 2 546 521**

Remarks:
The file contains technical information submitted after the application was filed and not included in this specification

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/13, A61K 2300/00;**
**A61K 31/4045, A61K 2300/00**

## Description

The invention relates to the field of the chemical-pharmaceutical industry, specifically to a novel melatonin and memantine combined preparation.

Memantine (or 3,5-dimethyladamantane-1-amine) is an adamantane derivative of the following structure:

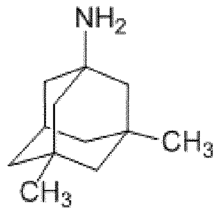

As a non-competitive antagonist of N-methyl-O-aspartate (NMDA)-receptors, it has a modulatory effect on the glutamatergic system. It regulates ion transport, blocks calcium channels, normalizes membrane potential, improves the transmission of a nervous impulse. It has nootropic, cerebrovasodilatoric, antihypoxic and psychoactive effects, improves cognitive processes, increases activities of daily living, reduces the fatiguability and symptoms of depression, reduces spasticity caused by brain diseases and injuries [*Community register of 15 medicinal products: http://wwwarlsnet.ru/mnn index id 2206. html*].

Melatonin (or N-acetyl-5-methoxytryptamine) is a synthetic epiphysis hormone analog of the following structure:

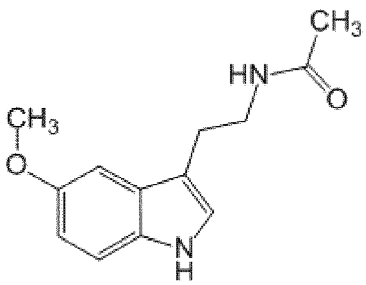

Under physiological conditions, melatonin secretion is increased soon after nightfall came, reaches a maximum at 2 to 4 a.m., and decreases during the second half of the night. The content of endogenous melatonin decreases with age. Melatonin controls circadian rhythms and a day-night cycle perception, has a sedative effect and improves falling asleep, adapts the body to quick change of time zones, reduces the stress responses, exhibits immunostimulatory and significant antioxidant properties, suppresses the secretion of gonadotropins and, in a lesser extent, other adenohypophysis hormones - corticotrophin, thyrotropin, and somatotropin [*Community register of medicinal products: http://www.rlsnet.ru/mnn index id 2278.htm*]. The use of a memantine and melatonin composition effective against disorders in patients having brain pathologies for the prevention, management, and therapy of amyloid intoxication signs is known from the prior art [*Patent* RU2536270 *Cl. publ. 12/20/2014]*. The prior art reference shows that the memantine and melatonin combination has a synergistic effect to improve cognitive dysfunction and reduce the concentration of beta-amyloid protein aggregates, which clumps form amyloid plaques in the brain of patients having Alzheimer's disease. A pharmaceutical composition containing a combination of memantine and melatonin can be provided in a tablet, capsule, injectable form, etc., and may further comprise pharmaceutically acceptable excipients.

However, the prior art reference discloses no particular embodiments of pharmaceutical compositions containing a combination of memantine and melatonin, furthermore, the pharmaceutically acceptable excipients are described in general terms and without any preferred ones.

An orally dissolving formulations comprising at least one water soluble polymer and memantine in amount of from 2.5 to 40 mg is known from the prior art [*Application* WO 2008005534 A2*, publ. 01/10/2008]*. This composition provides reliable delivery and absorption of the active ingredient (memantine), while also providing a dosing regimen that increases patient compliance. The prior art reference shows that the orally dissolving formulations may be used to treat various conditions, but is particularly suited to treat childhood behavioral disorders, such as autistic spectrum disorders or combined type Attention-Deficit/Hyperactivity Disorder (ADHD) and also to treat elderly patients suffering from Alzheimer's disease.

An orally disintegrating tablet comprising 3 parts by weight of melatonin, from 6 to 16 parts by weight of a disintegrator, from 10 to 22 parts by weight of an effervescing agent, from 40 to 70 parts by weight of a filling agent, from 3 to 6 parts by weight of an odor corrective and from 1 to 3 parts by weight of a lubricating agent is also known

from the prior art [*Application* CN 101143135 A*, publ. 03/19/2008].* The drug can be promptly disintegrated inside the mouth without water and is especially applicable for patients with deglutition difficulty or to treat elderly patients, children or patients under anesthesia.

However, these references do not disclose orally dissolving combination of memantine and melatonin.

The closest analog (the prior art) of the present invention is a pharmaceutical composition in a solid dosage form *[Patent* RU2488388 C1*, publ. 07/27/2013; Application No.* 14702015060746 A1*, publ. 30/04/2015]* containing a combination of memantine and melatonin, and pharmaceutically acceptable excipients in the following component ratio, wt%:

| | |
|---|---|
| Memantine | 40.0 to 90.0 |
| Melatonin | 2.0 to 5.0 |
| Diluent | 2.0 to 50.0 |
| Binder | 3.5 to 10.0 |
| Disintegrating agent | 1.5 to 10.0 |
| Antifriction agent | 0.2 to 3.0 |

The diluent is selected from lactose, starch, starch derivative, microcrystalline cellulose, sucrose, inverted sugar, dextrose, and dextrate; the disintegrating agent is selected from sodium carboxymethyl-cellulose, croscarmellose, pregelatinized starch; the binder is selected from polyvinylpyrrolidone, gelatin, cellulose derivatives, natural gums, polyethylene glycols, sodium alginate; the antifriction agent is selected from stearic acid and/or salts thereof, colloidal silicon dioxide, talc, sodium benzoate, sodium acetate, and sodium oleate.

As disadvantageous features, it may be noted that said reference discloses no stability and dissolution rate study results obtained for the provided pharmaceutical composition in an orally disintegrating tablet form

The object of the invention is to broaden the range of pharmaceutical compositions containing a combination of memantine and melatonin and to prepare pharmaceutical compositions having improved properties compared to the prior art.

The technical result of the present invention is to improve safety, storage stability and dissolution rate of a pharmaceutical composition containing a combination of memantine and melatonin.

The present invention object and technical result are achieved by a novel pharmaceutical composition in an orally disintegrating tablet form for the prevention and treatment of mental, behavioral, cognitive disorders, the composition comprising:

- from 2.0 to 15.0 wt% of memantine;
- from 1.5 to 10.0 wt% of melatonin;
- from 75.0 to 96.5 wt% of excipients.

In a preferred embodiment of the invention, the pharmaceutical composition comprises:

- from 2.0 to 15.0 wt% of memantine;
- from 1.5 to 10.0 wt% of melatonin, and
- from 65.0 to 80.0 wt% of a vehicle, and/or
- from 4.0 to 15.0 wt% of a disintegrating agent, and/or
- from 1.0 to 2.0 wt% of a binder, and/or
- from 1.0 to 2.5 wt% of a sweetener, and/or
- from 0.5 to 1.0 wt% of a glidant, and/or
- from 1.5 to 3.0 wt% of a flavoring agent.

The vehicle is preferably selected from, but not limited to, mannitol or a mannitol and copovidone mixture.

The disintegrating agent is preferably selected from, but not limited to, crospovidone, sodium carboxymethylcellulose, croscarmellose, pregelatinized starch.

The binder is preferably selected from, but not limited to, sorbitol, polyvinylpyrrolidone, gelatin, cellulose derivatives, natural gums, polyethylene glycols, sodium alginate.

The composition may optionally include a sweetener. The sweetener is selected from, but not limited to, maltitol, sodium saccharinate or mixtures thereof, sucralose, potassium acesulfame.

The glidant is selected from, but not limited to, colloidal silicon dioxide, stearic acid 5 and/or salts thereof, talc or mixtures thereof, sodium benzoate, sodium acetate, and sodium oleate.

As the flavoring agent, any agents commonly used in the preparation of pharmaceutical compositions in a tablet

form can be used. Preferably, the flavoring agent is selected from mint, menthol, strawberry, orange or lemon ones. This selection of the agents is preferred, but not limited to the above options.

The tablet is an orally disintegrating tablet.

In yet another embodiment, memantine is in an amount of 2.5 to 10.0 wt%, preferably 2.5 wt%, and melatonin is in an amount of 1.5 to 6.0 wt%, preferably 1.5 wt%.

In yet another embodiment, memantine and melatonin may be in amounts that include any of the above subranges, i.e. memantine may be, but is not limited to, in the amount ranging from, e.g., 2.5 to 10.0 wt%, or 2.5 to 9.5 wt%, or 2.5 to 9.0 wt%, 2.5 to 8.5 wt%, 2.5 to 8.0 wt%, 2.5 to 7.5 wt%, 2.5 to 7.0 wt%, 2.5 to 6.5 wt%, 2.5 to 6.0 wt%, 2.5 to 5.5 wt%, 2.5 to 5.0 wt%, 2.5 to 4.5 wt%, 2.5 to 4.0 wt%, 2.5 to 3.5 wt%, 2.5 to 3.0 wt%, and melatonin may be, but is not limited to, in the amount ranging from, e.g., 1.5 to 6.0 wt%, 1.5 to 5.5 wt%, 1.5 to 5.0 wt%, 1.5 to 4.5 wt%, 1.5 to 4.0 wt%, 1.5 to 3.5 wt%, 1.5 to 3.0 wt%, 1.5 to 2.5 wt%, 1.5 to 2.0 wt%.

The inventive pharmaceutical compositions are used for the prevention and treatment of mental, behavioral, cognitive disorders and impairments, including dementia of varying severity. The agent can be used for medical conditions associated with clinical signs of organic psychosyndrome (and it's the most critical component - dementia) as follows: Alzheimer's disease, vascular (multi-infarct) dementia, mild cognitive impairment, alcoholism, intracranial volumetric processes - tumors, subdural hematomas and brain abscesses, anoxia, craniocerebral trauma, normal pressure hydrocephalus, Parkinson's disease, Huntington's disease, progressive supranuclear paralysis, Pick's disease, amyotrophic lateral sclerosis, spinocerebellar degeneration, ophthalmoplegia associated with metachromatic leukodystrophy (adult form), Gellervorden-Spatz disease, hashish psychosis, advanced, infection, Creutzfeldt-Jacob disease, viral encephalitis, progressive multifocal leukoencephalopathy, neurosyphilis, Behcet's disease, chronic bacterial and fungal meningitis; deficiency conditions, Wernicke-Korsakoff syndrome, thiamine deficiency, vitamin B12 deficiency, folic acid deficiency, vitamin B3 deficiency, pellagra; metabolic disorders, dialysis dementia, hypo- and hyperthyroidism, severe renal failure, Cushing syndrome, hepatic failure, parathyroid disease, systemic lupus erythematosus and other collagen depended diseases associated with cerebral vasculitis, multiple sclerosis, Whipple's disease.

A method for preparing said pharmaceutical composition in a tablet form comprises weighing and sieving the starting materials, mixing and tableting thereof. Preferably, the tableting is performed using the direct compression technique. Finished tablets can be packaged in blisters, and blisters can be packaged in cardboard boxes.

While the invention herein has been described by means of various typical practical methods of application, it should be noted that these practical embodiments describe only the concepts of the present invention and its application. Those skilled in the art would appreciate that numerous modifications could be made in typical embodiments in practicing the invention without departing from the scope of the invention.

In addition, it should be appreciated that various features and/or characteristics of various practices of the invention may be combined. Therefore, it should be understood that, in addition to the described practice embodiments, numerous modifications may be made thereto, and other methods of application may be developed without departing from the scope of the invention.

In addition, other embodiments of practicing the invention may come to mind to those skilled in the art after reading the present specification and practicing the invention provided herein. The specification sections and examples are intended only for illustration of a typical practice in view of the knowledge in the art and guidance provided herein.

The present invention can be illustrated by the following examples.

Examples provide pharmaceutical compositions in a tablet form containing a combination of memantine and melatonin, which are illustrative and by no way limiting the scope of the present invention.

**Example 1**. Preparation of Composition Variants

The preparation of pharmaceutical compositions in a tablet form comprises weighing and sieving the starting materials, mixing and tableting thereof using the direct compression technique.

The following composition variants were prepared as shown in Tables 1-5 below.

Table 1. Variants 1 to 3.

| **Formulation** | **Amount of material, wt%** | | |
|---|---|---|---|
| | **Variant 1** | **Variant 2** | **Variant 3** |
| Memantine (in hydrochloride form) | 2.5 | 8.0 | 15.0 |
| Melatonin | 1.5 | 6.0 | 10.0 |
| Mannitol | 75.46 | 69.8 | 60 |
| Copovidone | 0.175 | 2.5 | 5 |

(continued)

| Formulation | Amount of material, wt% | | |
|---|---|---|---|
| | Variant 1 | Variant 2 | Variant 3 |
| Crospovidone | 13.05 | 9.0 | 4.0 |
| Sorbitol | 1.68 | 1.4 | 1.0 |
| Maltitoi | 1.235 | 0.9 | 0.5 |
| Sodium saccharinate | 1.0 | 0.7 | 0.5 |
| Colloidal silicon dioxide | 0.9 | 0.7 | 0.5 |
| Mint/menthol flavoring agent | 2.5 | 1.0 | 1.5 |

Table 2. Variants 4 to 6.

| Formulation | Amount of material, wt% | | |
|---|---|---|---|
| | Variant 4 | Variant 5 | Variant 6 |
| Memantine (in hydrochloride form) | 2.5 | 8.0 | 15.0 |
| Melatonin | 1.5 | 6.0 | 10.0 |
| Mannitol | 75.46 | 69.8 | 60 |
| Copovidone | 0.175 | 2.5 | 5 |
| Crospovidone | 13.05 | 9.0 | 4.0 |
| Sorbitol | 1.68 | 1.4 | 1.0 |
| Maltitoi | 1.235 | 0.9 | 0.5 |
| Stearic acid | 1.0 | 0.7 | 0.5 |
| Magnesium stearate | 0.9 | 0.7 | 0.5 |
| Strawberry flavoring agent | 2.5 | 1.0 | 1.5 |

Table 3. Variants 7 to 9.

| Formulation | Amount of material, wt% | | |
|---|---|---|---|
| | Variant 7 | Variant 8 | Variant 9 |
| Memantine (in hydrochloride form) | 2.5 | 8.0 | 15.0 |
| Melatonin | 1.5 | 6.0 | 10.0 |
| Mannitol | 77.56 | 70.8 | 63.5 |
| Copovidone | 0.175 | 2.5 | 5 |
| Crospovidone | 13.05 | 9.0 | 4.0 |
| Sorbitol | 1.68 | 1.4 | 1.0 |
| Sodium saccharinate | 1.235 | 0.9 | 0.5 |
| Talc | 0.9 | 0.7 | 0.5 |
| Lemon flavoring agent | 1.4 | 0.7 | 0.5 |

Table 4. Variants 10-12.

| Formulation | Amount of material, wt% | | |
|---|---|---|---|
| | Variant 10 | Variant 11 | Variant 12 |
| Memantine (in hydrochloride form) | 2.5 | 8.0 | 15.0 |
| Melatonin | 1.5 | 6.0 | 10.0 |
| Mannitol | 75.46 | 69.8 | 60 |
| Copovidone | 0.175 | 2.5 | 5 |
| Sodium carboxymethyl-cellulose | 13.05 | 9.0 | 4.0 |
| Polyethylene glycol | 1.68 | 1.4 | 1.0 |
| Maltitol | 1.235 | 0.9 | 0.5 |
| Sucralose | 1.0 | 0.7 | 0.5 |
| Colloidal silicon dioxide | 0.9 | 0.7 | 0.5 |
| Mint/menthol flavoring agent | 2.5 | 1.0 | 1.5 |

Table 5. Variants 13-15.

| Formulation | Amount of material, wt% | | |
|---|---|---|---|
| | Variant 13 | Variant 14 | Variant 15 |
| Memantine (in hydrochloride form) | 2.5 | 8.0 | 15.0 |
| Melatonin | 1.5 | 6.0 | 10.0 |
| Mannitol | 75.46 | 69.8 | 60 |
| Copovidone | 0.175 | 2.5 | 5 |
| Croscarmellose | 13.05 | 9.0 | 4.0 |
| Sodium alginate | 1.68 | 1.4 | 1.0 |
| Maltitol | 1.235 | 0.9 | 0.5 |
| Acesulfame | 1.0 | 0.7 | 0.5 |
| Colloidal silicon dioxide | 0.9 | 0.7 | 0.5 |
| Mint/menthol flavoring agent | 2.5 | 1.0 | 1.5 |

**Example 2.** Determination of Tablet Stability by Accelerated Ageing Technique.

Storage stability of tablets of variants I to 15 was evaluated based on the content of melatonin and was compared to the stability of tablets of the prior art *[Patent* RU 2488388 C1*, publ. 07/27/2013. Example 1].*

Prior Art Memantine and Melatonin Tablet Formulation.

| Material | mg | Wt% |
|---|---|---|
| Memantine | 100 | 40,00% |
| Melatonin | 5 | 2,00% |
| Lactose | 70 | 28,00% |
| Microcrystalline cellulose | 24.75 | 9,90% |
| Starch | 26 | 10,40% |
| Povidone | 12.5 | 5,00% |
| Croscarmellose | 9.75 | 3,90% |
| Calcium stearate | 2 | 0,80% |
| Total tablet weight | 250 | 100,00% |

Tablets of variants I to 15 and of the prior art were packed into aluminum foil blisters ('Alu-Alu') and placed into a stability chamber under accelerated test conditions. Melatonin content was analyzed by HPLC using standards.

"Accelerated aging" technique involves maintaining the test medicine at temperatures and humidity above the temperature and humidity of its storage during handling. At elevated temperatures, physicochemical processes proceeding in medicines are usually accelerated resulting in undesirable quality changes over time. Thus, at elevated temperature, a period of time while the controlled quality measures in the medicine remain within the acceptable range (experimental shelf life) is artificially reduced compared to a shelf life at a storage temperature. This allows significantly reduce the time required to determine the shelf life. Based on the results obtained in the process of medicine "accelerated aging," it is also 15 possible to solve the inverse problem, i.e., to determine the storage temperature that guarantees any desired shelf life.

The shelf life (S) at the storage temperature (tst) is related to the experimental shelf life (Sexp) at an elevated temperature of the experimental storage (texp) by the following relationship:

$$S = K \cdot S_{exp}$$

$$K = A\frac{t_{exp} - t_{st}}{10}$$

wherein the coefficient of concordance is

The temperature coefficient of the chemical reaction rate (A) is assumed to be 2.5. The above relationship is based on the Vant-Hoff rule about 2 to 4 fold increase in the rates of chemical reactions with an increase in temperature by 10 $^0$ C.

In accordance with the GM 1.1.0009.15, the value of the coefficient of concordance (K) depending on the selected temperature interval ($t_{exp}$-$t_{st}$), which is equal to 30 $^0$ C, is 15.6. The experimental storage period with the selected shelf life of 3 years is 71 days.

Statistical processing the parameters and the presentation of the study results were performed in compliance with the Rules of Good Clinical Practice of the Eurasian Economic 10 Union using SPSS Statistics 19.0 statistical software suite.

After 71 days of storage under the accelerated aging technique conditions, tablets of variants 1 to 15 were found to have equivalent stability and remained chemically pure. Tablets of the prior art remained chemically pure for less than 10 days, further the melatonin content decreased by more than 3%. In other words, tablets ofvariants I to 15 of the present invention are much more stable compared to the prior art.

Table 6. Stability evaluation in tablet variants 1 to 15 during storage using accelerated aging technique compared to the prior art

| Storage time at ($t_{exp}$-$t_{st}$) = 30 °C, days | Melatonin content in % based on theoretical content as measured by HPLC | | | | | | | | | | | | | | | |
|---|---|---|---|---|---|---|---|---|---|---|---|---|---|---|---|---|
| | Composition variant number according to Example 1 | | | | | | | | | | | | | | | The prior art |
| | 1 | 2 | 3 | 4 | 5 | 6 | 7 | -8 | 9 | 10 | 11 | 12 | 13 | 14 | 15 | |
| 0 | 100 | 100 | 100 | 100 | 100 | 100 | 100 | 100 | 100 | 100 | 100 | 100 | 100 | 100 | 100 | 100 |
| 10 | 100 | 100 | 100 | 100 | 100 | 100 | 100 | 100 | 100 | 100 | 100 | 100 | 100 | 100 | 100 | 99.9 |
| 20 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 99.5 |
| 30 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 99.1 |
| 40 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 98.4 |
| 50 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 98.0 |
| 60 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 97.2 |
| 71 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 96.8 |

Table 7. Stability evaluation in tablet variants 1 to 15 during storage for 12 months compared to the prior art

| Storage time at 25 °C and relative humidity of 75%, months | Melatonin content in % based on theoretical content as measured by HPLC | | | | | | | | | | | | | | | |
|---|---|---|---|---|---|---|---|---|---|---|---|---|---|---|---|---|
| | Composition variant number according to Example 1 | | | | | | | | | | | | | | | The prior art |
| | 1 | 2 | 3 | 4 | 5 | 6 | 7 | 8 | 9 | 10 | 11 | 12 | 13 | 14 | 15 | |
| 0 | 100 | 100 | 100 | 100 | 100 | 100 | 100 | 100 | 100 | 100 | 100 | 100 | 100 | 100 | 100 | 100 |
| 1 | 100 | 100 | 100 | 100 | 100 | 100 | 100 | 100 | 100 | 100 | 100 | 100 | 100 | 100 | 100 | 99.9 |
| 3 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 99.7 |
| 6 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 99.3 |
| 9 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 98.6 |
| 12 | 99.9 | 99.9 | 99.9 | 99.9 | 99.9 | 99.9 | 99.9 | 99.9 | 99.9 | 99.9 | 99.9 | 99.9 | 99.9 | 99.9 | 99.9 | 98.2 |

Table 8. Evaluation of dissolution kinetics in tablet variants 1 to 15 compared to the prior art

| Time, sec | Memantine amount passed into solution, in % based on the maximum theoretical concentration | | | | | | | | | | | | | | | |
|---|---|---|---|---|---|---|---|---|---|---|---|---|---|---|---|---|
| | Composition variant number according to Example 1 | | | | | | | | | | | | | | | The prior art |
| | 1 | 2 | 3 | 4 | 5 | 6 | 7 | 8 | 9 | 10 | 11 | 12 | 13 | 14 | 15 | |
| 0 | 0 | 0 | 0 | 0 | 0 | 0 | 0 | 0 | 0 | 0 | 0 | 0 | 0 | 0 | 0 | 0 |
| 10 | 17.9 | 17.7 | 17.6 | 17.3 | 17.6 | 17.7 | 18.2 | 17.5 | 17.3 | 17.6 | 17.5 | 17.6 | 17.8 | 17.6 | 18.0 | 18.0 |
| 30 | 44.9 | 44.8 | 44.7 | 44.5 | 44.7 | 44.8 | 44.9 | 44.8 | 44.6 | 44.8 | 44.7 | 44.2 | 44.9 | 44.8 | 44.7 | 38.8 |
| 60 | 61.2 | 61.1 | 60.8 | 60.0 | 60.8 | 60.0 | 61.1 | 60.8 | 60.0 | 60.8 | 60.0 | 60.0 | 60.8 | 60.9 | 60.8 | 45.6 |
| 120 | 100 | 100 | 100 | 100 | 100 | 100 | 100 | 100 | 100 | 100 | 100 | 100 | 100 | 100 | 100 | 53.0 |
| 300 | 100 | 100 | 100 | 100 | 100 | 100 | 100 | 100 | 100 | 100 | 100 | 100 | 100 | 100 | 100 | 76.4 |
| 600 | 100 | 100 | 100 | 100 | 100 | 100 | 100 | 100 | 100 | 100 | 100 | 100 | 100 | 100 | 100 | 100 |

**Example 3.** Tablet Stability Determination using Long Time Test Technique

Storage stability of tablets of variants 1 to 15 was evaluated based on the content of melatonin and was compared to the stability of tablets of the prior art [*Patent* RU 2488388 C1*, publ. 07/27/2013, Example 1*].

Prior Art Memantine and Melatonin Tablet Formulation.

| Material | mg | Wt% |
|---|---|---|
| Memantine | 100 | 40,00% |
| Melatonin | 5 | 2,00% |
| Lactose | 70 | 28,00% |
| Microcrystalline cellulose | 24.75 | 9,90% |
| Starch | 26 | 10,40% |
| Povidone | 12.5 | 5,00% |
| Croscarmellose | 9.75 | 3,90% |
| Calcium stearate | 2 | 0,80% |
| Total tablet weight | 250 | 100,00% |

Tablets of variants 1 to 15 and of the prior art were packed into aluminum foil blisters ('Alu-Alu') and placed into a stability chamber under long time test conditions. Melatonin content was analyzed by HPLC using standards.

After 12 months of storage under the real-time conditions, tablets of variants 1 to 15 were found to have equivalent stability and remained chemically pure. Tablets of the prior art remained chemically pure for less than 6 months, further the melatonin content decreased by more than 0.7%. In other words, tablets of variants 1 to 15 of the present invention are more stable compared to the prior art.

Inventors suggest that the unexpected effect of improving the stability of orally disintegrating dosage forms of variants 1 to 15 compared to the prior art may be due to decreasing the memantine content in solid dosage forms. High content (more than 40 wt%) of memantine, which is alkali by its chemical nature, in solid dosage forms of the prior art may have a destabilizing effect on the second active component of the dosage form - melatonin, which content was determined in the following examples.

**Example 4.** Comparison of Dissolution Profiles in Tablets of Variants I to 15 and of Prior Art.

Dissolution kinetics in tablets of variants 1 to 15 was evaluated based on the content of the substance and compared to dissolution kinetics in tablets of the prior art [*Patent* RU 2488388 C1*, publ. 07/27/2013, Example 1*].

Prior Art Memantine and Melatonin Tablet Formulation.

| Material | mg | Wt% |
|---|---|---|
| Memantine | 100 | 40,00% |
| Melatonin | 5 | 2,00% |
| Lactose | 70 | 28,00% |
| Microcrystalline cellulose | 24.75 | 9,90% |
| Starch | 26 | 10,40% |
| Povidone | 12.5 | 5,00% |
| Croscarmellose | 9.75 | 3,90% |
| Calcium stearate | 2 | 0,80% |
| Total tablet weight | 250 | 100,00% |

A device for determining the dissolution rate is a three-necked 1 L flask. A thermometer is introduced into one of the necks, a glass tube for sampling and sample integrating is introduced into the other one, and the main part of the device, i.e. a cylindrical stainless steel basket of 3.6 cm high and 2.5 cm in diameter as a grid with openings of 40 meshes in diameter (about 0.351 mm) is introduced into the third one. The basket is mounted onto the motor shaft.

A solvent medium is added into the flask (1000 mL), in this experiment, it is an alkaline solution of pancreatin corresponding to human saliva medium and important for determining the solubility of sublingual (orodispersible) dosage forms. The test sample is placed into the cylindrical basket, which is installed at a distance of 2 cm from the bottom of the flask.

During the experiment, the temperature of the dissolution medium is maintained constant ($37 \pm 0.5\,^{0}$C). Basket

rotation rate in the medium is adjusted within ± 5% and is 200 rpm. At predetermined time points, 1 to 2 mL specimens are sampled to determine the content of the solute. The sampled volume of the solvent is immediately replenished with the new one.

Dissolution is controlled based on the selected component, i.e. memantine. For comparative samples, 250 mg tablets of variants 1 to 15 and of the prior art were used to provide, once completely dissolved, the solution comprising 6.25 mg/L, 20 mg/L, 37.5 mg/L of memantine for variants 1 to 15, and 100 mg/L of memantine for the prior art.

The obtained results in a percentage of the dissolved active ingredient by the maximum concentration are shown in Table 8.

The results demonstrate that the dissolution rate of tablets of variants 1 to 15 of the present invention is significantly higher than the dissolution rate of tablets of the prior art, in particular time to 50% dissolution of tablets of variants 1 to 15 of the present invention is twice less than for the comparative sample of the prior art, which can be particularly useful for the preparation of orodispersible tablets.

The obtained results show that the selected excipient formulation and ratios are optimal for the orally disintegrating formulations containing a combination of memantine and melatonin.

**Example 5.** Acute Toxicity Study of Preparation

To study the novel composition acute toxicity, the following composition variants were further prepared as shown in Table 9 below.

Table 9. Variants 16 to 17.

| **Formulation** | **Substance amount, wt. %** | |
|---|---|---|
| | **Variant 16** | **Variant 17** |
| Memantine (in hydrochloride form) | 2.0 | 10.0 |
| Melatonin | 10.0 | 2.0 |
| Mannitol | 67.8 | 68.7 |
| Copovidone | 0.2 | 5.6 |
| Crospovidone | 12.9 | 8.7 |
| Sorbitol | 1.5 | 1.2 |
| Maltitol | 1.2 | 0.8 |
| Sodium saccharinate | 1.0 | 0.7 |
| Colloidal silicon dioxide | 0.9 | 0.6 |
| Mint/menthol flavoring agent | 2.5 | 1.7 |

Test Group Characteristics

In the acute toxicity study, groups of 10 male Wistar rats were formed for each dose.

Rats were intragastrically administered with compositions of variants 1, 16, and 17, and of the prior art [*patent* RU 2488388 C1*, publ. 07/27/2013, Example 1*].

Doses needed to calculate LD50 were determined experimentally. $LD_{50}$ was calculated using the probit analysis according to Prozorovsky's methodology.

Lethal Dose Determination

In the acute toxicity study, doses were selected experimentally, doses were calculated by memantine as a more toxic component.

Groups, doses, and results of the acute toxicity study for the compositions of variants 1, 16 and 17, and of the prior art are provided in Table 10.

Table 10. $LD_{50}$ values for rats in intragastric administration

| Group No. | Active ingredient dosage (by memantine), mg/kg | Composition | Frequency of administration | LD50 with confidence interval, mg/kg |
|---|---|---|---|---|
| 1 | 100 | | | |
| 2 | 300 | | | |
| 3 | 500 | Variant 1 | once | 509.5 (358.8 to 648.2) |
| 4 | 600 | | | |
| 5 | 800 | | | |
| 6 | 100 | Variant 16 | once | 498.1 (242.9 to 553.1) |
| 7 | 300 | | | |
| 8 | 500 | | | |
| 9 | 600 | | | |
| 10 | 800 | | | |
| 11 | 100 | | | |
| 12 | 300 | | | |
| 13 | 500 | Variant 17 | once | 469.4 (320.6 to 507.4) |
| 14 | 600 | | | |
| 15 | 800 | | | |
| 16 | 100 | | | |
| 17 | 300 | | | |
| 18 | 500 | The prior art | once | 168.0 (142.9 to 353.1) |
| 19 | 600 | | | |
| 20 | 800 | | | |

Analysis of the acute toxicity study results for a composition of substances prepared in the ratio corresponding to variant 1, 16 and 17 and in the ratio obtained for the prior art showed the composition of substances administered intragastrically exhibits toxic properties in dosages allowing to range it according to Hodge and Sterner's classification to substances having the $3^{d}$ toxic level - moderately toxic. According to K. K. Sidorov's classification, the combination of substances administered abdominally belongs to the $4^{th}$grade - low-toxic substances.

Compositions of variants 1, 16 and 17 were significantly less toxic than the composition of the prior art administered with the same memantine dosage. Without wishing to be bound by any particular theory, inventors believe that this effect may be associated with the optimal memantine to melatonin ratio in the compositions of the invention.

Based on the active ingredient content in the test lozenges, the toxicometry results, observations of experimental animals for 14 days after acute administration and necropsy data, the inventive compositions administered intragastrically (in finished dosage form) could be classified as $5^{th}$ grade practically non-toxic drugs (II Hodge et all Clinical Toxicology of Commercial Products. Acute Poisoning. Ed. IV, Baltimore, 1975, 427 p.; K.K. Sidorov, 1977).

**Example 6.** Multiple Dose Toxicity

A study of chronic (180-day) toxicity was performed using outbred Wistar rats (90 males and 90 females, 180 rats in total). In the chronic toxicity study, animals were assigned to 9 groups of 20 animals per group (10 females and 10 males). The preparations were administered intragastrically.

A chronic toxicity data analysis showed that comparison groups (the inventive compositions of variants 1, 16, 17 and of the prior art) exhibited a sporadic mortality that was probably associated with the development of cardiopulmonary decompensation. The animal body weight values, feed, and water consumption were within the physiological norm.

Test inventive compositions of variants 1, 16 and 17 had no adverse impact on the animal behavior.

The frequency of disturbances in the heart bioelectrical activity when administered test inventive compositions

of variants 1, 16, 17 corresponds to the frequency of similar disturbances resulted from the drug memantine administration that is indicating of the absence of the potentiation effect between the components of the inventive compositions of variants 1, 16, 17.

The administration of the inventive composition of variants 1, 16, 17 has no adverse impact on hematopoiesis. The recorded significant deviations over control (leukocytes, monocytes, erythrocytes, hemoglobin, hematocrit, MCV, MCH, platelets) were within the physiological norm. There are no toxic effect potentiations between pharmaceutical substances in test inventive compositions of variants 1, 16, 17.

The hemostatic profile was within the physiological norm.

In the biochemical blood analysis, there was no evident relationship between the obtained results and other measures that was indicative of the functional nature of the determined deviations causing no significant cytolytic phenomena for this regimen and duration of drug administration, as confirmed by histological studies. There were no potentiations between the components (memantine + melatonin).

The urinalysis showed that the 180-day administration of the inventive compositions of variants 1, 16, 17 resulted in the protein and blood cell appearance in the urine. The changes were persistent. The obtained results are consistent with those previously found during clinical examinations, and the described events of urine discoloration and the appearance of urethrorrhea. Histological examination showed no pathological processes in kidneys. Therefore, the experimental data show that the damage was not associated with the kidney structure and functional state. There was no potentiation effect between the components (memantine + melatonin).

The study of the internal organ histomorphological structure revealed no signs of pathological changes after administration of the inventive compositions of variants 1, 16, 17 and of the prior art.

There was no local irritative effect.

The results of the performed studies concerning the general toxic properties of the preparation suggest that there is no toxic effect potentiation between the components of the inventive compositions of variants 1, 16, 17.

Performed studies concerning general toxic properties of the novel inventive compositions of variants 1, 16, 17 suggest that there is no toxic effect potentiation between the components of the combination preparation.

The invention can be used in medicine, chemistry, pharmaceutical industry.

## Claims

1. A pharmaceutical composition in an orally disintegrating tablet form for the prevention and treatment of mental, behavioral, cognitive disorders, wherein the composition comprises:

    - from 2.0 to 15.0 wt% of memantine;
    - from 1.5 to 10.0 wt% of melatonin;
    - from 75.0 to 96.5 wt% of pharmaceutically acceptable excipients.

2. The pharmaceutical composition in an orally disintegrating tablet form according to claim 1, **characterized in that** the composition comprises:

    - from 2.0 to 15.0 wt% of memantine;
    - from 1.5 to 10.0 wt% of melatonin, and
    - from 65.0 to 80.0 wt% of a vehicle comprising at least one component selected from mannitol and copovidone, and/or
    - from 4.0 to 15.0 wt% ofa disintegrating agent comprising at least one component selected from crospovidone, sodium carboxymethyl-cellulose, croscarmellose and pregelatinized starch, and/or
    - from 1.0 to 2.0 wt% of a binder comprising at least one component selected from sorbitol, polyvinylpyrrolidone, gelatin, cellulose derivatives, natural gums, polyethylene glycols and sodium alginate, and/or
    - from 1.0 to 2.5 wt% of a sweetener comprising at least one component selected from maltitol and sodium saccharinate, and/or
    - from 0.5 to 1.0 wt% of a glidant comprising at least one component selected from colloidal silicon dioxide, stearic acid and/or salts thereof, talc or mixtures thereof, stearic acid and/or salts thereof, colloidal silicon dioxide, talc, sodium benzoate, sodium acetate, and sodium oleate, and/or
    - from 1.5 to 3.0 wt% of a flavoring agent.

3. A pharmaceutical composition according to claim 2, **characterized in that** the composition comprises:

    - from 2.0 to 15.0 wt% ofmemantine;

- from 1.5 to 10.0 wt% of melatonin;
- from 65.0 to 80.0 wt% of a vehicle which is a mannitol and copovidone mixture;
- from 4.0 to 15.0 wt% ofa disintegrating agent which is crospovidone;
- from 1.0 to 2.0 wt% of a binder which is sorbitol;
- from 1.0 to 2.5 wt% of a sweetener selected from maltitol, sodium saccharinate or a mixture thereof;
- from 0.5 to 1.0 wt% of a glidant selected from colloidal silicon dioxide, stearic acid, magnesium stearate, talc, or mixtures thereof;
- from 1.5 to 3.0 wt% of a flavoring agent.

**4.** The pharmaceutical composition according to claim 3, **characterized in that** the flavoring agent is selected from mint, menthol, strawberry, orange or lemon ones.

**5.** The pharmaceutical composition according to claim 3, **characterized in that** the vehicle is a mannitol and copovidone mixture, the sweetener is a maltitol and sodium saccharinate mixture, the glidant is colloidal silicon dioxide, the flavoring agent is selected from mint or menthol ones.

**6.** The pharmaceutical composition according to claim 3, **characterized in that** the composition comprises memantine in an amount of 2.5 to 10.0 wt%.

**7.** The pharmaceutical composition according to claim 6, **characterized in that** the composition comprises memantine in an amount of 2.5 wt%.

**8.** The pharmaceutical composition according to claim 3, **characterized in that** the composition comprises melatonin in an amount of 1.5 to 6.0 wt%.

**9.** The pharmaceutical composition according to claim 8, **characterized in that** the composition comprises melatonin in an amount of 1.5 wt%.

**10.** A pharmaceutical composition according to claim 3, **characterized in that** the composition comprises, wt%

| | |
|---|---|
| Memantine (in hydrochloride form) | 2.5% |
| Melatonin | 1.5% |
| Mannitol | 75.46% |
| Copovidone | 0.175% |
| Crospovidone | 13.05% |
| Sorbitol | 1.68% |
| Maltitol | 1.235% |
| Sodium saccharinate | 1.0% |
| Mint/menthol flavoring agent | 2.5%. |

**11.** The pharmaceutical composition according to any one of claims 1-10, **characterized in that** the composition is used for the prevention and treatment of cognitive impairment in dementia of varying severity.

**12.** A method for preparing the pharmaceutical composition in an orally disintegrating tablet form according to any one of claims 1-11 comprising weighing and sieving the starting materials, mixing and tableting thereof.

**13.** The method for preparing according to claim 12, **characterized in that** the tableting is performed using the direct compression technique.

**Patentansprüche**

**1.** Pharmazeutische Zusammensetzung in Form einer sich im Mund auflösenden Tablette zur Vorbeugung und Behandlung von mentalen, verhaltensbedingten, kognitiven Störungen, wobei die Zusammensetzung Folgendes umfasst:

- von 2,0 Gew.-% bis 15,0 Gew.-% Memantin;
- von 1,5 Gew.-% bis 10,0 Gew.-% Melatonin;
- von 75,0 Gew.-% bis 96,5 Gew.-% pharmazeutisch verträgliche Hilfsstoffe.

**2.** Pharmazeutische Zusammensetzung in Form einer sich im Mund auflösenden Tablette nach Anspruch 1, **dadurch gekennzeichnet, dass** die Zusammensetzung Folgendes umfasst:

- von 2,0 Gew.-% bis 15,0 Gew.-% Memantin;
- von 1,5 Gew.-% bis 10,0 Gew.-% Melatonin, und
- von 65,0 Gew.-% bis 80,0 Gew.-% eines Vehikels, das mindestens eine Komponente umfasst, die aus Mannit und Copovidon ausgewählt ist, und/oder
- von 4,0 Gew.-% bis 15,0 Gew.-% eines Sprengmittels, das mindestens eine Komponente umfasst, die aus Crospovidon, Natriumcarboxymethylcellulose, Croscarmellose und vorgelatinierter Stärke ausgewählt ist, und/oder
- von 1,0 Gew.-% bis 2,0 Gew.-% eines Bindemittels, das mindestens eine Komponente umfasst, die aus Sorbit, Polyvinylpyrrolidon, Gelatine, Cellulosederivaten, natürlichen Gummis, Polyethylenglycolen und Natriumalginat ausgewählt ist, und/oder
- von 1,0 Gew.-% bis 2,5 Gew.-% eines Süßungsmittels, das mindestens eine Komponente umfasst, die aus Maltit und Natriumsaccharin ausgewählt ist, und/oder
- von 0,5 Gew.-% bis 1,0 Gew.-% eines Gleitmittels, das mindestens eine Komponente umfasst, die aus kolloidalem Siliciumdioxid, Stearinsäure und/oder Salzen davon, Talk oder Mischungen davon, Stearinsäure und/oder Salzen davon, kolloidalem Siliciumdioxid, Talk, Natriumbenzoat, Natriumacetat und Natriumoleat ausgewählt ist, und/oder
- von 1,5 Gew.-% bis 3,0 Gew.-% eines Aromastoffes.

**3.** Pharmazeutische Zusammensetzung nach Anspruch 2, **dadurch gekennzeichnet, dass** die Zusammensetzung Folgendes umfasst:

- von 2,0 Gew.-% bis 15,0 Gew.-% Memantin;
- von 1,5 Gew.-% bis 10,0 Gew.-% Melatonin;
- von 65,0 Gew.-% bis 80,0 Gew.-% eines Vehikels, das eine Mischung aus Mannit und Copovidon ist;
- von 4,0 Gew.-% bis 15,0 Gew.-% eines Sprengmittels, das Crospovidon ist;
- von 1,0 Gew.-% bis 2,0 Gew.-% eines Bindemittels, das Sorbit ist;
- von 1,0 Gew.-% bis 2,5 Gew.-% eines Süßungsmittels, das aus Maltit, Natriumsaccharin oder einer Mischung davon ausgewählt ist;
- von 0,5 Gew.-% bis 1,0 Gew.-% eines Gleitmittels, das aus kolloidalem Siliciumdioxid, Stearinsäure, Magnesiumstearat, Talk oder Mischungen davon ausgewählt ist;
- von 1,5 Gew.-% bis 3,0 Gew.-% eines Aromastoffes.

**4.** Pharmazeutische Zusammensetzung nach Anspruch 3, **dadurch gekennzeichnet, dass** der Aromastoff aus Minze, Menthol, Erdbeere, Orange oder Zitrone ausgewählt ist.

**5.** Pharmazeutische Zusammensetzung nach Anspruch 3, **dadurch gekennzeichnet, dass** das Vehikel eine Mischung aus Mannit und Copovidon ist, das Süßungsmittel eine Mischung aus Maltit und Natriumsaccharin ist, das Gleitmittel kolloidales Siliciumdioxid ist, der Aromastoff aus denen Minze oder Menthol ausgewählt ist.

**6.** Pharmazeutische Zusammensetzung nach Anspruch 3, **dadurch gekennzeichnet, dass** die Zusammensetzung Memantin in einer Menge von 2,5 Gew.-% bis 10,0 Gew.-% umfasst.

**7.** Pharmazeutische Zusammensetzung nach Anspruch 6, **dadurch gekennzeichnet, dass** die Zusammensetzung Memantin in einer Menge von 2,5 Gew.-% umfasst.

**8.** Pharmazeutische Zusammensetzung nach Anspruch 3, **dadurch gekennzeichnet, dass** die Zusammensetzung Melatonin in einer Menge von 1,5 Gew.-% bis 6,0 Gew.-% umfasst.

**9.** Pharmazeutische Zusammensetzung nach Anspruch 8, **dadurch gekennzeichnet, dass** die Zusammensetzung Melatonin in einer Menge von 1,5 Gew.-% umfasst.

**10.** Pharmazeutische Zusammensetzung nach Anspruch 3, **dadurch gekennzeichnet, dass** die Zusammensetzung Folgendes in Gew.-% umfasst

| | |
|---|---|
| Memantin (in Hydrochloridform) | 2,5 % |
| Melatonin | 1,5 % |
| Mannit | 75,46 % |
| Copovidon | 0,175 % |
| Crospovidon | 13,05 % |
| Sorbit | 1,68 % |
| Maltit | 1,235 % |
| Natriumsaccharin | 1,0 % |
| Minz-/Menthol-Aromastoff | 2,5 %. |

**11.** Pharmazeutische Zusammensetzung nach einem der Ansprüche 1-10, **dadurch gekennzeichnet, dass** die Zusammensetzung zur Vorbeugung und Behandlung von kognitiver Beeinträchtigung bei Demenz unterschiedlichen Schweregrades verwendet wird.

**12.** Verfahren zur Herstellung der pharmazeutischen Zusammensetzung in Form einer sich im Mund auflösenden Tablette nach einem der Ansprüche 1-11, umfassend Wiegen und Sieben der Ausgangsmaterialien, deren Mischen und Tablettieren.

**13.** Verfahren zur Herstellung nach Anspruch 12, **dadurch gekennzeichnet, dass** das Tablettieren unter Verwendung der Direktpresstechnik durchgeführt wird.

**Revendications**

**1.** Composition pharmaceutique sous la forme d'un comprimé à désintégration orale destinée à la prévention et au traitement de troubles mentaux, comportementaux, cognitifs, ladite composition comprenant :

- de 2,0 à 15,0 % en poids de mémantine ;
- de 1,5 à 10,0 % en poids de mélatonine ;
- de 75,0 à 96,5 % en poids d'excipients pharmaceutiquement acceptables.

**2.** Composition pharmaceutique sous la forme d'un comprimé à désintégration orale selon la revendication 1, **caractérisée en ce que** la composition comprend :

- de 2,0 à 15,0 % en poids de mémantine ;
- de 1,5 à 10,0 % en poids de mélatonine, et
- de 65,0 à 80,0 % en poids d'un véhicule comprenant au moins un constituant choisi parmi le mannitol et la copovidone, et/ou
- de 4,0 à 15,0 % en poids d'un agent délitant comprenant au moins un constituant choisi parmi la crospovidone, la carboxyméthylcellulose sodique, la croscarmellose et l'amidon prégélatinisé, et/ou
- de 1,0 à 2,0 % en poids d'un liant comprenant au moins un constituant choisi parmi le sorbitol, la polyvinylpyrrolidone, la gélatine, les dérivés de cellulose, les gommes naturelles, les polyéthylèneglycols et l'alginate de sodium, et/ou
- de 1,0 à 2,5 % en poids d'un édulcorant comprenant au moins un constituant choisi parmi le maltitol et le saccharinate de sodium, et/ou
- de 0,5 à 1,0 % en poids d'un agent de glissement comprenant au moins un constituant choisi parmi le dioxyde de silicium colloïdal, l'acide stéarique et/ou ses sels, le talc ou ses mélanges, l'acide stéarique et/ou ses sels, le dioxyde de silicium colloïdal, le talc, le benzoate de sodium, l'acétate de sodium et l'oléate de sodium, et/ou
- de 1,5 à 3,0 % en poids d'un agent aromatisant.

**3.** Composition pharmaceutique selon la revendication 2, **caractérisée en ce que** la composition comprend :

- de 2,0 à 15,0 % en poids de mémantine ;
- de 1,5 à 10,0 % en poids de mélatonine ;
- de 65,0 à 80,0 % en poids d'un véhicule qui est un mélange de mannitol et de copovidone ;
- de 4,0 à 15,0 % en poids d'un agent délitant qui est la crospovidone ;
- de 1,0 à 2,0 % en poids d'un liant qui est le sorbitol ;
- de 1,0 à 2,5 % en poids d'un édulcorant choisi parmi le maltitol, le saccharinate de sodium ou un mélange de ceux-ci ;
- de 0,5 à 1,0 % en poids d'un agent de glissement choisi parmi le dioxyde de silicium colloïdal, l'acide stéarique, le stéarate de magnésium, le talc ou leurs mélanges ;
- de 1,5 à 3,0 % en poids d'un agent aromatisant.

**4.** Composition pharmaceutique selon la revendication 3, **caractérisée en ce que** l'agent aromatisant est choisi parmi les agents aromatisants de la menthe, du menthol, de la fraise, de l'orange ou du citron.

**5.** Composition pharmaceutique selon la revendication 3, **caractérisée en ce que** le véhicule est un mélange de mannitol et de copovidone, l'édulcorant est un mélange de maltitol et de saccharinate de sodium, l'agent de glissement est le dioxyde de silicium colloïdal, l'agent aromatisant est choisi parmi les agents aromatisants de la menthe ou du menthol.

**6.** Composition pharmaceutique selon la revendication 3, **caractérisée en ce que** la composition comprend de la mémantine en une quantité de 2,5 à 10,0 % en poids.

**7.** Composition pharmaceutique selon la revendication 6, **caractérisée en ce que** la composition comprend de la mémantine en une quantité de 2,5 % en poids.

**8.** Composition pharmaceutique selon la revendication 3, **caractérisée en ce que** la composition comprend de la mélatonine en une quantité de 1,5 à 6,0 % en poids.

**9.** Composition pharmaceutique selon la revendication 8, **caractérisée en ce que** la composition comprend de la mélatonine en une quantité de 1,5 % en poids.

**10.** Composition pharmaceutique selon la revendication 3, **caractérisée en ce que** la composition comprend, en % en poids

| | |
|---|---|
| Mémantine (sous forme de chlorhydrate) | 2,5 % |
| Mélatonine | 1,5 % |
| Mannitol | 75,46 % |
| Copovidone | 0,175 % |
| Crospovidone | 13,05 % |
| Sorbitol | 1,68 % |
| Maltitol | 1,235 % |
| Saccharinate de sodium | 1,0 % |
| Agent aromatisant de menthe/menthol | 2,5 %. |

**11.** Composition pharmaceutique selon l'une quelconque des revendications 1 à 10, **caractérisée en ce que** la composition est utilisée pour la prévention et le traitement de troubles cognitifs dans la démence de gravité variable.

**12.** Procédé de préparation de la composition pharmaceutique sous la forme d'un comprimé à désintégration orale selon l'une quelconque des revendications 1 à 11, comprenant le pesage et le tamisage des matières premières, leur mélange et leur pastillage.

**13.** Procédé de préparation selon la revendication 12, **caractérisé en ce que** le pastillage est réalisé à l'aide de la technique de compression directe.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- RU 2536270
- WO 2008005534 A2
- CN 101143135 A
- RU 2488388 C1
- WO 14702015060746 A1


### Non-patent literature cited in the description


- Clinical Toxicology of Commercial Products. **II HODGE.** Acute Poisoning. 1975, 427